# EUROPÄISCHE PATENTANMELDUNG

(11) **EP 4 431 017 A1**
(43) Veröffentlichungstag der Anmeldung: **18.09.2024**
(21) Anmeldenummer: 24159193.2
(22) Anmeldetag: 22.02.2024
(51) Int. Cl.: A61B 5/259, A61B 5/268, A61N 1/04, C09J 9/02

(54) **TROCKENELEKTRODENVORRICHTUNG**

(30) Priorität: 07.03.2023 DE 102023000860
(71) Anmelder: Lohmann GmbH & Co. KG, 56567 Neuwied (DE)
(72) Erfinder: Lutz, Volker, 56579 Rengsdorf (DE); Weisenstein, Christian, 57548 Kirchen (DE)

(57) **Zusammenfassung**

Die Erfindung geht aus von einer Trockenelektrodenvorrichtung mit zumindest einer elektrisch leitenden Anschlusseinheit und mit einer, insbesondere elektrisch leitenden, Klebstoffschichteinheit, welche eine Kontaktfläche zur Kontaktierung einer Haut aufweist und welche in einem Anwendungszustand eine elektrische Energieübertragung zwischen der Anschlusseinheit und der Haut bereitstellt.

Um eine Konstruktion zu verbessern, wird vorgeschlagen, dass bei einer Draufsicht eine Konturfläche der Anschlusseinheit eine Konturfläche der Kontaktfläche zumindest zu 80 % überlappt.

## Beschreibung

Die Erfindung betrifft eine Trockenelektrodenvorrichtung nach dem Anspruch 1.

Traditionelle Gelelektroden haben den Nachteil, dass diese mit der Zeit austrocknen und deshalb für Langzeitanwendungen nicht geeignet sind. Trockenelektroden teilen diesen Nachteil zwar nicht, dennoch besteht nach wie vor das Bedürfnis nach einer Trockenelektrode für Langzeitanwendungen, die sich preislich nicht wesentlich von den anderen Elektrodentypen unterscheidet und über die Anwendungsdauer weder ihre Haftung verliert noch Hautirritationen auslöst.

Trockenelektroden und elektrisch leitende Klebstoffe zur Herstellung dieser sind beispielsweise aus der WO 2020/178217 A1 bekannt. Der offenbarte Klebstoff weist eine ionische Flüssigkeit auf, um eine ausreichende elektrische Leitfähigkeit zu gewährleisten. Dies wird damit begründet, dass das Bereitstellen einer elektrischen Leitfähigkeit nur durch elektrisch leitende Partikel dazu führen würde, dass dem Klebstoff so viele elektrisch leitende Partikel hinzugefügt werden müssten, dass die Haftung des Klebstoffs hierdurch beeinträchtigt werden würde. Ferner weisen die offenbarten Elektroden elektrisch leitende Schichten auf, welche zwischen der elektrisch leitenden Klebstoffschicht und einer Anschlusseinheit angeordnet sind und beispielsweise aus Silber/Silberchlorid bestehen können. Diese elektrisch leitenden Schichten vergrößern und/oder verschieben den Bereich, innerhalb welchem Signale durch den elektrisch leitenden Klebstoff an eine Anschlusseinheit, üblicherweise ein metallischer Druckknopf; weitergeleitet werden können. Dies steigert zwar die Signalqualität und die Designfreiheit, erhöht aber sowohl die Materialkosten als auch die Komplexität des Herstellungsverfahrens,

Die Aufgabe der Erfindung besteht insbesondere darin, eine gattungsgemäße Vorrichtung bereitzustellen, welche verbesserte Eigenschaften bezüglich einer Konstruktion aufweist. Die Aufgabe wird erfindungsgemäß durch die Merkmale des Anspruchs 1 gelöst, während vorteilhafte Ausgestaltungen und Weiterbildungen der Erfindung den Unteransprüchen entnommen werden können.

Die Erfindung geht aus von einer Trockenelektrodenvorrichtung mit zumindest einer elektrisch leitenden Anschlusseinheit und mit einer, insbesondere elektrisch leitenden, Klebstoffschichteinheit, welche eine Kontaktfläche zur Kontaktierung einer Haut aufweist und welche in einem Anwendungszustand zur Bereitstellung einer elektrischen Energieübertragung zwischen der Anschlusseinheit und der Haut vorgesehen ist.

Es wird vorgeschlagen, dass sich bei einer Draufsicht eine Konturfläche der Anschlusseinheit eine Konturfläche der Kontaktfläche zumindest zu 80 % überlappt. Hierdurch ist die effektive Hautfläche, über die Signale an die Anschlusseinheit weitergeleitet werden können, deutlich geringer als in aus dem Stand der Technik bekannten Trockenelektroden, welche beispielsweise Silber/Silberchlorid-Schichten benutzen, um die Kontaktfläche auf eine doppelte oder sogar dreifache Größe der Anschlusseinheit zu erweitern. Es wurde aber überraschenderweise festgestellt, dass die Signalqualität auch bei kleinen Kontaktflächen für viele Anwendungen noch ausreichend ist. Dies erhöht eine Designfreiheit und erlaubt eine günstigere und einfachere Herstellung der Trockenelektrodenvorrichtung, da insbesondere auf zusätzliche elektrisch leitende Schichten und elektrisch leitende Klebstoffschichten, welche weit über die Anschlusseinheit hinausragen, verzichtet werden kann.

Es ist möglich, dass die Trockenelektrodenvorrichtung einen Teil eines Trockenelektrodensystems ausbildet, wobei das Trockenelektrodensystem eine oder mehrere Trockenelektrodenvorrichtungen und zusätzliche funktionelle Bauteile wie Gehäuse, Kabel, Anschlüsse, Tragegurte oder -bänder, Displays, Prozessoren und/oder Kommunikationsgeräte aufweist. Bevorzugt ist die Trockenelektrodenvorrichtung als eine Einweg-Trockenelektrode ausgebildet, welche dazu vorgesehen ist, auf die Haut aufgeklebt und nach Abschluss einer Verwendungsdauer entsorgt zu werden. Hierdurch kann eine kompakte und kostengünstige Konstruktion der Tfrockenelektrodenvorrichtung erreicht werden.

Es wäre denkbar, dass die Trockenelektrodenvorrichtung eine Mehrzahl an Anschlusseinheiten und/oder Klebstoffschichteinheiten aufweist. In diesem Fall könnte entweder jeweils eine der Anschlusseinheiten mittels einer der Klebstoffschichteinheiten eine elektrische Energieübertragung zwischen der Haut und der Anschlusseinheit bereitstellten, oder eine Mehrzahl von Anschlusseinheiten könnte mittels einer einzigen Klebstoffschichteinheit eine elektrische Energieübertragung zwischen der Haut und der Anschlusseinheit bereitstellen. Vorzugsweise weist die Trockenelektrodenvorrichtung genau eine Anschlusseinheit und genau eine Klebstoffschichteinheit auf Vorstellbar wäre, dass die Trockenelektrodenvorrichtung zusätzlich zur Anschlusseinheit und Klebstoffschichteinheit zumindest eine Sensoreinheit aufweist, welche dazu vorgesehen ist, Biosignale zu erfassen. Die Sensoreinheit könnte die erfassten Biosignale entweder ebenfalls an die Anschlusseinheit oder an eine separate weitere Anschlusseinheit weiterleiten. Beispielsweise könnte die Sensoreinheit zumindest einen optischen Sensor, insbesondere einen PPG-Sensor, zumindest einen Wärmesensor, zumindest einen pH-Sensor und/oder zumindest einen Feuchtigkeitssensor aufweisen.

Es ist möglich, dass die Trockenelektrodenvorrichtung zumindest eine Abdeckeinheit aufweist. Die Abdeckeinheit ist insbesondere dazu vorgesehen, in dem Anwendungszustand gemeinsam mit der Klebstoffschichteinheit eine Fixierung der Trockenelektrodenvorrichtung relativ zu der Haut bereitzustellen. Vorzugsweise weist die Abdeckeinheit einen Träger, welcher beispielsweise als ein Textil oder ein Zellstoff ausgebildet sein könnte, und einen haft klebrigen Hautklebstoff, welcher auf dem Träger aufgebracht ist, auf. Bevorzugt ist die Abdeckeinheit mit der Anschlusseinheit kraft- und/oder formschlüssig verbunden. Besonders bevorzugt umschließt bei der Draufsicht eine Kontur der Abdcckcinhcit eine Kontur der Klebstoffschichteinheit, wobei die Abdeckeinheit in dem Anwendungszustand innerhalb des umschließenden Teilbereichs der Kontur eine Klebeverbindung mit der Haut bereitstellt.

Eine Größe der Anschlusseinheit, Klebstoffschichteinheit und Abdeckeinheit ist insbesondere von einem Anwendungsbereich der Trockenelektrodenvorrichtung abhängig. Vorzugsweise weist eine Konturfläche der Abdeckeinheit bei der Draufsicht einen Flächeninhalt von höchstens 100 cm², besonders bevorzugt höchstens 75 cm², und von vorzugsweise mindestens 8 cm², besonders bevorzugt mindestens 12 cm², auf. Vorzugsweise weist die Konturfläche der Klebstoffschichteinheit bei der Draufsicht einen Flächeninhalt von höchstens 40 cm², besonders bevorzugt höchstens 30 cm², und von vorzugsweise mindestens 2 cm², besonders bevorzugt mindestens 4 cm², auf.

Vorteilhaft weist die Trockenelektrodenvorrichtung zumindest einen Release Liner auf. Der Release Liner bedeckt vorzugsweise eine der Anschlusseinheit abgewandte Seite der Klebstoffschichteinheit und ist dazu vorgesehen, vor einer Applikation der Trockenelektrodenvorrichtung entfernt zu werden.

Die Anschlusseinheit kann beliebige, elektrisch leitende Materialien aufweisen. "Elektrisch leitende Materialien" sollen in diesem Zusammenhang als sämtliche Materialien, welche bei 25°C eine elektrische Leitfähigkeit von mindestens 10⁶ S/m aufweisen, insbesondere Metalle und Kohlenstoff. Es wäre möglich, dass die Anschlusseinheit vollständig aus elektrisch leitenden Materialien besteht. Alternativ könnte die Anschlusseinheit eine Beschichtung aus einem oder mehreren elektrisch leitenden Materialien aufweisen. Ferner könnte die Anschlusseinheit elektrisch leitende Partikel, welche ein oder mehrere elektrisch leitende Materialien aufweisen, enthalten.

Die Anschlusseinheit weist vorteilhaft ein Kontaktelement auf, welches an einer der Klebstoffschichteinheit zugewandten Seite der Anschlusseinheit angeordnet und dazu vorgesehen ist, in dem Anwendungszustand eine elektrische Energieübertragung zwischen der Haut und der Anschlusseinheit bereitzustellen. Bevorzugt weist das Kontaktelement eine Kontaktplatte auf, deren Länge und Breite der einem Fünffachen, vorteilhaft einem Zehnfachen, besonders vorteilhaft einem Fünfzehnfachen ihrer Dicke entspricht. Besonders bevorzugt weist die Kontaktplatte eine, der Klebstoffschichteinheit zugewandte, ebene Oberfläche auf. Die Kontaktplatte kann das Kontaktelement insbesondere vollständig ausbilden, alternativ könnte die Kontaktplatte lediglich einen Teilbereich des Kontaktelements ausbilden. Es ist denkbar, dass die Klebstoffschichteinheit eine homogene Dicke aufweist und das Kontaktelement auf der Klebstoffschichteinheit aufliegt. Alternativ könnte die Klebstoffschichteinheit im Bereich des Kontaktelements eine geringere Dicke aufweisen und das Kontaktelement teilweise oder vollständig in die Klebstoffschichteinheit eingebettet sein.

Besonders vorteilhaft weist die Anschlusseinheit ein Anschlusselement auf, welches an einer der Klebstoffschichteinheit abgewandten Seite der Anschlusseinheit angeordnet und dazu vorgesehen ist, in dem Anwendungszustand mit einer externen Einheit verbunden zu werden. Das Anschlusselement kann den Anschluss an die externe Einheit mittels beliebiger, insbesondere reversibler, form- und/oder kraftschlüssiger Verbindungsarten bereitstellen, beispielsweise könnte das Anschlusselement als ein Rastelement, Klammerelement, Gewindeelement, Hakenelement und/oder Schwalbenschwanzelement ausgebildet sein. Vorteilhaft ist das Anschlusselement manuell an die externe Einheit anschließbar. Besonders vorteilhaft bilden das Kontaktelement und das Anschlusselement gemeinsam ein Druckknopfelement aus. Die externe Einheit kann beispielsweise der Weiterleitung und/oder Auswertung von Biosignalen und/oder der Bereitstellung von elektrischen Impulsen, beispielsweise zur Stimulierung von Muskelgruppen, dienen.

Es wäre möglich, dass das Anschlusselement und das Kontaktelement als Teilbereiche eines zusammenhängenden Bauteils ausgebildet sind. Alternativ könnten die Anschlusseinheit zusätzlich zu dem Anschlusselement und Kontaktelement ein Verbindungselement aufweisen, welches das Anschlusselement und Kontaktelement aneinander fixiert. Das Verbindungselement könnte das Anschlusselement und Kontaktelement mittels einer, insbesondere reversiblen, form- und/oder kraftschlüssigen Verbindung aneinander fixieren und als ein Rastelement, Klammerelement, Gewindeelement, Hakenelement und/oder Schwalbenschwanzelement ausgebildet sein. Alternativ könnte das Verbindungselement das Anschlusselement und Kontaktelement mittels einer irreversiblen, formschlüssigen Verbindung aneinander fixieren und als eine Niete oder ein Klebemittel ausgebildet sein. Vorzugsweise weisen das Anschlusselement und Kontaktelement korrespondierende Teilbereiche auf, welche gemeinsam das Anschlusselement und Kontaktelement aneinander fixieren. Die Teilbereiche könnten gemein eine, insbesondere reversible, form- und/oder kraftschlüssige Verbindung, beispielsweise eine Rastverbindung, Klammerverbindung, Drehverbindung, Hakenverbindung und/oder Schwalbenschwanzverbindung ausbilden. Bevorzugt bilden die Teilbereiche gemeinsam eine irreversible, formschlüssige Verbindung, beispielsweise einer Nietverbindung, Klebeverbindung und/oder Schweißverbindung aus.

Die Klebstoffschichteinheit kann insbesondere eine beliebige Anzahl an Klebstoffschichten aufweisen, welche entweder direkt aufeinander oder auf einen Träger aufgebracht sein können. Insbesondere weist die Klebstoffschichteinheit wenigstens eine Klebstoffschicht auf. Es wäre denkbar, dass die Klebstoffschichten der Klebstoffschichteinheit Ausnehmungen und/oder eine sich lokal ändernde Dicke aufweisen. Bevorzugt sind sämtliche Klebstoffschichten der Klebstoffschichteinheit als zusammenhängende Schichten mit einer Dicke, welche, mit Ausnahme einer Verjüngung im Bereich des Kontaktelements, falls dieses in die Klebstoffschichteinheit eingebettet ist, jeweils in sich homogen ist.

Unter einer "Kontaktfläche der Klebstoffschichteinheit" soll eine Teiloberfläche einer der Anschlusseinheit abgewandten Oberfläche der Klebstoffschichteinheit verstanden werden, wobei in dem Anwendungszustand Biosignale von dem mit der Kontaktfläche kontaktierten Teil der Haut mittels einer elektrischen Energieübertragung an das Kontaktelement geleitet werden. Es wäre denkbar, dass die elektrische Energieübertragung eine kapazitive Energieübertragung und das Kontaktelement als eine kapazitive Elektrode ausgebildet ist, wobei die Klebstoffschichteinheit in dem Anwendungszustand lediglich eine kapazitive elektrische Kopplung zwischen der Anschlusseinheit und der Haut bereitstellt. In diesem Fall ist die Klebstoffschichteinheit vorzugsweise frei von elektrisch leitenden Materialien, wobei die Kontaktfläche abhängig von einer Größe des Kontaktelements und eines Abstands des Kontaktelements von der Haut in dem Anwendungszustand ist.

Vorteilhaft ist die elektrische Energieübertragung eine elektrisch leitende Energieübertragung und das Kontaktelement in dem Anwendungszustand elektrisch leitend mit der Haut verbunden, wobei die Klebstoffschichteinheit in dem Anwendungszustand eine direkte elektrische Leitung zwischen der Anschlusseinheit und der Haut bereitstellt. In diesem Fall kann die Klebstoffschichteinheit isotrop elektrisch leitend sein, wodurch die Kontaktfläche und die der Anschlusseinheit abgewandte Oberfläche der Klebstoffschichteinheit zueinander identisch sind. Bevorzugt ist die Klebstoffschichteinheit anisotrop elektrisch leitend, wodurch die Größe der Kontaktfläche identisch zu der der Klebstoffschichteinheit zugewandten Oberfläche des Kontaktelements ist.

Unter dem "Anwendungszustand" ist in diesem Zusammenhang ein Zustand zu verstehen, in welchem die Trockenelektrodenvorrichtung an der Haut angebracht und funktionsbereit ist. Insbesondere ist das Anschlusselement in dem Anwendungszustand an die externe Einheit angeschlossen, bevorzugt misst die Trockenelektrodenvorrichtung in dem Anwendungszustand Biosignale, insbesondere ein EKG-Signal, und übermittelt diese an die externe Einheit zur Auswertung.

Unter der "Draufsicht" soll eine Betrachtung der Trockenelektrodenvorrichtung verstanden werden, deren Blickrichtung parallel zu einer Flächennormalen der Klebstoffschichteinheit ausgerichtet ist Unter einer "Konturfläche" eines Elements oder einer Einheit soll eine Fläche verstanden werden, welche in der Draufsicht durch eine Kontur des Elements oder der Einheit eingeschlossen wird. Die Kontur eines Elements oder einer Einheit ist als der kleinstmögliche geschlossene Rand zu verstehen, welcher das Element oder die Einheit als Ganzes umschließt. Vorzugsweise ist eine Kontur der Anschlusseinheit identisch zu einer Kontur des Kontaktelements, insbesondere einer Kontur der Kontaktplatte. Hierbei ist die Kontur eines Elements unabhängig von über oder unter dem Element angeordneten weiteren Elementen zu betrachten, entsprechend ist eine Draufsicht der Trockenelektrodenvorrichtung davon unabhängig, ob die Trockenelektrodenvorrichtung von oben oder von unten betrachtet wird.

Es ist vorstellbar, dass sich die Kontur der Klebstoffschichteinheit und die Kontur der Anschlusseinheit bezüglich einer Form voneinander unterscheiden. Bevorzugt ist die Kontur der Klebstoffschichteinheit formidentisch zu der Kontur der Anschlusseinheit ausgebildet. Besonders bevorzugt sind die Kontur der Klebstoffschichteinheit und die Kontur der Anschlusseinheit zueinander konzentrisch angeordnet. Die Kontur der Klebstoffschichteinheit und die Kontur der Anschlusseinheit können insbesondere beliebige Formen aufweisen, beispielsweise können die Konturen dem Umfang eines Rechteck, insbesondere eines Quadrats, eines Ovals, insbesondere eines Kreises, oder einer beliebigen anderen, aus Bögen und geraden Linien zusammengesetzten, Form entsprechen.

Es wäre möglich, dass die Trockenelektrodenvorrichtung elektrisch leitende Schichten aufweist, welche zwischen der Klebstoffschichteinheit und der Anschlusseinheit angeordnet sind. Um eine Konstruktion der Trockenelektrodenvorrichtung weiter zu vereinfachen, wird vorgeschlagen, dass die Klebstoffschichteinheit unmittelbar an der Anschlusseinheit und insbesondere an dem Kontaktelement angeordnet ist. Darunter, dass die Klebstoffschicht "unmittelbar" an der Anschlusseinheit angeordnet ist, soll verstanden werden, dass die Klebstoffschichteinheit und das Kontaktelement frei von elektrisch leitenden Beschichtungen, Trägern oder Abdeckungen sind, welche teilweise oder vollständig zwischen der Klebstoffschichteinheit und dem Kontaktelement angeordnet sind. Hierdurch kann der Verzicht auf eine Kontaktfläche, welche deutlich größer als das Kontaktelement ist, dazu genutzt werden, Materialkosten und Hcrstcllungsschrittc einzusparen.

Es wäre denkbar, dass die Konturfläche der Klebstoffschichteinheit kleiner als oder identisch zu der Konturfläche der Anschlusseinheit ist. In diesem Fall wäre es notwendig, dass die Trockenelektrodenvorrichtung zumindest eine zusätzliche Schicht aufweist, welche in dem Anwendungszustand zwischen der Haut und der Anschlusseinheit angeordnet ist und in der Draufsicht eine Konturfläche aufweist, welche größer als die Konturfläche der Anschlusseinheit ist, um eine direkte elektrische Kontaktierung der Haut durch die Anschlusseinheit zu verhindern. Alternativ könnte die Konturfläche der Klebstoffschichteinheit um ein Vielfaches größer als die Konturfläche der Anschlusseinheit sein, wodurch beispielsweise auf die Abdcckeinhcit verzichtet werden könnte. Um eine Konstruktion der Trockenelektrodenvorrichtung zu vereinfachen, wird vorgeschlagen, dass die Klebstoffschichteinheit vorzugsweise anisotrop elektrisch leitend ist und bei der Draufsicht die Konturfläche der Klebstoffschichteinheit höchstens 100 %, vorteilhaft höchstens 75 %, besonders vorteilhaft höchstens 50 % und besonders bevorzugt höchstens 25 % größer als eine Konturfläche der Anschlusseinheit ist. Vorteilhaft beträgt bei der Draufsicht ein minimaler Abstand einer Kontur der Klebstoffschichteinheit von einer Kontur der Anschlusseinheit an jeder Stelle mindestens 1 mm, vorteilhaft mindestens 2 mm, wodurch eine direkte elektrische Kontaktierung der Haut durch die Anschlusseinheit vermieden werden kann. Hierdurch können Klebstoff und, falls eine Abdeckeinheit vorhanden ist, Abdeckmaterial eingespart werden. Vorteilhaft kann ein Hautbereich, welcher von der Trockenelektrodenvorrichtung abgedeckt wird, reduziert werden, wodurch auch bei gleichbleibender Atmungsaktivität der Klebstoffschichteinheit eine Irritation der Haut vermieden und ein Tragekomfort erhöht werden kann.

Um eine Konstruktion der Trockenelektrodenvorrichtung weiter zu vereinfachen, wird vorgeschlagen, dass die Klebstoffschichteinheit dazu vorgesehen ist, in dem Anwendungszustand eine Fixierung der Trockenelektrodenvorrichtung relativ zu der Haut vollständig bereitzustellen. Insbesondere ist die Trockenelektrodenvorrichtung frei von Abdeckeinheiten, welche in dem Anwendungszustand zu einer Fixierung der Trockenelektrodenvorrichtung relativ zu der Haut beitragen. Hierdurch können sowohl Materialkosten als auch Herstelhingsschritte eingespart werden. Vorteilhaft kann eine noch kompaktere Konstruktion der Trockenelektrodenvorrichtung erreicht werden, wodurch ein Tragekomfort weiter erhöht werden kann.

Um eine Konstruktion der Trockenelektrodenvorrichtung weiter zu verbessern, wird vorgeschlagen, dass die Klebstoffschichteinheit eine Klebstoffschicht aufweist, welche ein Polymer-Netzwerk mit zumindest einem Acrylatcopolymer und zumindest eine Art von elektrisch leitenden Partikeln aufweist und welche frei von ionischen Flüssigkeiten ist. Unter einem "Polymer-Netzwerk" soll eine übergeordnete Struktur verstanden werden, welche aus miteinander verknüpften Polymeren besteht. Es wäre denkbar, dass das Polymer-Netzwerk mehrere, zueinander unterschiedlich ausgebildete Acrylatcopolymere aufweist. Bevorzugt weist das Polymer-Netzwerk genau eine Art von Acrylatcopolymer auf. Das Acrylatcopolymer kann insbesondere als ein statistisches Copolymer, ein Block-Copolymer oder als ein Homo-Polymer ausgebildet sein. Analog zur Anschlusseinheit können die elektrisch leitenden Partikel der Klebstoffschicht ebenfalls beliebige elektrisch leitende Materialien aufweisen. Die elektrisch leitenden Partikel könnten vollständig aus den elektrisch leitenden Materialien bestehen. Beispielsweise könnten die elektrisch leitenden Partikel aus einem elektrisch leitfähigen Polymer wie PEDOT: PSS bestehen. Alternativ könnten die elektrisch leitenden Partikel mit elektrisch leitenden Materialien beschichtet sein. Beispielsweise könnten die elektrisch leitenden Partikel einen Kunststoff-, Aluminium- oder Glaskern aufweisen, welcher mit Kupfer, Silber oder anderen Metallen beschichtet ist Bevorzugt weist die Klebstoffschicht bei 25°C und bei einer Impedanzmessung senkrecht durch die Klebstoffschicht eine Impedanz von höchstens 50 Ω bei 10 Hz auf.

Vorteilhaft ist die Klebstoffschicht biokompatibel. Darunter, dass die Klebstoffschicht "biokompatibel" ist, soll verstanden werden, dass die Klebstoffschicht genormte Prüfungen zur Biokompatibilität von Stoffen besteht. Die genormten Prüfungen sind hierbei von einem Land, in welchem die Trockenelektrodenvorrichtung Anwendung findet, abhängig, bevorzugt besteht die Klebstoffschicht zumindest eine Prüfung auf Hautirritation gemäß ISO 10993-10, eine Prüfung auf Hautsensibilisierung gemäß ISO 10993-10 und eine Prüfung auf Zytotoxizität gemäß ISO 10993-5.

Vorzugsweise ist die Klebstoffschicht ist als eine Haftklebstoffschicht ausgebildet. Unter einer "Haftklebstoffschicht" soll eine Klebstoffschicht verstanden werden, welche bei 25°C und ohne einen vorhergehenden Aktivierungsschritt, wie beispielsweise eine Erwärmung oder Bestrahlung, permanent haftklebrig ist. Vorzugsweise ist die Klebstoffschicht frei von strukturellen Klebstoffen, welche dazu vorgesehen sind, nach einem Applizieren auszuhärten, wobei die strukturellen Klebstoffe vor dem Aushärten keine oder nur eine geringe Haftung aufweisen. Bevorzugt ist die Klebstoffschicht in dem Anwendungszustand unmittelbar an der Haut angeordnet. Besonders bevorzugt bildet die Klebstoffschicht die Klebstoffschichteinheit vollständig aus. Hierdurch kann eine Konstruktion der Trockenelektrodenvorrichtung weiter verbessert werden. Vorteilhaft kann durch die Verwendung eines elektrisch leitenden Acrylatklebsfoffs eine biokompatible und für Langzeithautanwendungen geeignete Klebstoffschicht bereitgestellt werden. Die bereits erwähnte überraschende Erkenntnis, dass auch eine geringere Signalqualität in vielen Anwendungen akzeptabel ist, führte dazu, dass getestet wurde, ob auf die aus dem Stand der Technik bekannten ionischen Flüssigkeiten verzichtet werden kann. Diese Experimente führten zu dem Ergebnis, dass Acrylatklebstoffe mit elektrisch leitenden Partikeln auch ohne ionische Flüssigkeiten oder elektrisch leitende Salze eine ausreichende Signalqualität gekoppelt mit einer Biokompatibilität und einer guten Langzeithaftung auf Haut aufweisen.

Darüber hinaus wird vorgeschlagen, dass das Acrylatcopolymer 70 bis 98,5 Gew.-%, vorteilhaft 85 bis 96 Gew.-%, besonders vorteilhaft 90 bis 95 Gew.-% an unpolaren Acrylatmonomeren und 1,5 bis 30 Gew.-%, vorteilhaft 3 bis 15 Gew.-%, besonders vorteilhaft 5 bis 10 Gew.-% an polaren Acrylatmonomeren aufweist, wobei sich die Gewichtsanteile insbesondere zu 100 % addieren. Die Begriffe "Acrylatcopolymer" und "Acrylatmonomer" sollen so verstanden werden, dass sie reine Acrylatcopolymere und Acrylatmonomere sowie Methacrylatcopolymere und Methacrylatmonomere umfassen. Darunter, dass ein Acrylatmonomer "polar" ist, soll in diesem Zusammenhang verstanden werden, dass das Acrylatmonomer zumindest eine polare Gruppe, wie beispielsweise eine OH-Gruppe, aufweist. Darunter, dass ein Acrylatmonomer "unpolar" ist, soll in diesem Zusammenhang verstanden werden, dass das Acrylatmonomer frei von polaren Gruppen ist. Insbesondere können beliebige, aus dem Stand der Technik bekannte, Acrylatmonomere verwendet werden, wie beispielsweise Acrylsäure, Methylacrylat, Ethylacrylat, n-Butylacylat, iso-Butylacrylat, tert-Butylacrylat, 2-Ethylhexylacrylat, 2-Hydroxyehtylacrylat, 2-Hydroxypropylacrylat, 2-Hydroxyethylacrylat, iso-Bomylacrylat, N,N-Dimethylaminoethylacrylat, Laurylacrylat, Stearylacrylat, Benzylacrylat, Methacrylsäure, Methylmethacrylat, Ethylmethacrylat, Isopropylacrylat, n-Butylmethacrylat, iso-Butylmetharcylat, tert-Butylmethacrylat und/oder Cyclohexylmethacrylat. Vorzugsweise besteht das Acrylatcopolymer im Wesentlichen aus den polaren und unpolaren Acrylatmonomeren. "Im Wesentlichen" ist in diesem Zusammenhang so zu verstehen, dass das Acrylatcopolymer zwar weitere Bestandteile, wie beispielsweise Reste eines Initiators, aufweisen kann, diese aber unter 0,5 Gew.-%, vorteilhaft unter 0,1 Gew.-% des Acrylatcopolymers ausbilden und die Eigenschaften des Acrylatpolymers nicht wesentlich beeinflussen. Hierdurch kann eine Konstruktion der Trockenelektrodenvorrichtung weiter verbessert werden. Vorteilhaft kann durch die sorgfähige Einstellung der Polarität des Acrylatcopolymers eine Atmungsaktivität der Klebstoffschicht optimiert werden. Eine zu geringe Atmungsaktivität führt zu einer Ansammlung von Feuchtigkeit zwischen der Trockcnelektrodenvorrichtung und der Haut, wodurch Hautirritationen entstehen und die Langzeithaftung der Trockenelektrodenvorrichtung reduziert wird. Eine zu hohe Atmungsaktivität führt zu einer Ansammlung von Feuchtigkeit in der Klebstoffschicht, wodurch die Langzeithaftung der Trockenelektrodenvorrichtung ebenfalls reduziert wird.

Die angegebenen Gewichtsanteile beziehen sich auf einen Anteil der jeweiligen Substanz an einer Klebstoffmasse, welche zur Herstellung der Klebstoffschicht beschichtet oder weitere, dem Fachmann bekannte, Additive aufweisen. Vorteilhaft weist die Klebstoffschicht mindestens 90 Gew.-%, vorteilhaft mindestens 95 Gew.-% an Substanzen des Acrylatcopolymers auf.

Die Formulierungen "X bis Y", "mindestens X", "höchstens X" sollen in diesem Zusammenhang so verstanden werden, dass die Grenzen des jeweiligen Wertebereichs eingeschlossen sind.

Es wäre denkbar, dass das Acrylatcopolymer frei von Acrylsäure ist und stattdessen ausschließlich andere Arten von polaren Acrylmonomeren, wie beispielsweise 2-Hydroxyethylacrylat, aufweist. Um eine Konstruktion der Trockenelektrodenvorrichtung weiter zu vereinfachen, wird vorgeschlagen, dass das Acrylatcopolymer 0,1 bis 10 Gew.-%, vorteilhaft 3 bis 7 Gew.-% an Acrylsäure aufweist. Das Zuordnen des Gewichtsanteils zu dem Acrylatcopolymer soll dahingehend verstanden werden, dass die bezeichneten Substanzen im Anschluss an die Polymerisationsreaktion einen Teil des Acrylatcopolymers ausbilden. Bevorzugt ist Acrylsäure das einzige polare Acrylatmonomer des Acrylatcopolymers. Hierdurch kann eine Glasübergangstemperatur des Acrylatcopolymers optimiert werden. Vorteilhaft kann aufgrund der hohen Glasübergangstemperatur der Acrylsäure von 100,85°C die Glasübergangstemperatur des Acrylatcopolymers nach oben korrigiert werden. Besonders vorteilhaft kann durch das Einstellen der Glasübergangstemperatur des Acrylatcopolymers eine Haftklebrigkeit und eine Kohäsion der

Klebstoffschicht optimiert werden, wodurch ein vorzeitiges Abfallen der Trockenelektrodenvorrichtung durch einen kohäsiven Bruch und ein Zurückbleiben von Rückständen auf der Haut vermieden werden kann. Weist die Klebstoffschicht eine zu hohe Kohäsion auf, kann sie aufgrund der Unebenheit von Hautoberflächen eine Benetzung der Haut nicht ausreichend bereitstellen. Weist die Klebstoffschicht eine zu geringe Kohäsion auf, ist die Trockenelektrodenvorrichtung nicht ausreichend fest an der Haut fixiert, wozu es zu Verschiebungen oder teilweisen bis vollständigen Ablösen der Trockenelektrodenvorrichtung kommen kann, ferner kann eine zu geringe Kohäsion dazu führen, dass die Klebstoffschicht bei einer Entfernung der Trockenelektrodenvorrichtung nach der Anwendungsdauer Rückstände auf der Haut zurücklässt.

Das Optimieren einer Eigenschaft des Acrylatcopolymers mittels einer bestimmten Auswahl von Acrylatmonomeren soll dahingehend verstanden werden, dass das Acrylatcopolymer, welches durch eine Polymerisationsreaktion entsteht, andere Eigenschaften aufweist als ein vergleichbares Acrylatcopolymer, welche durch dieselbe Polymerisationsreaktion entsteht und andere Acrylatmonomere beziehungsweise andere Gewichtsanteile der Acrylatmonomere aufweist. Das die Polymerisationsreaktionen identisch sind, soll dahingesehen verstanden werden, dass eine Anzahl an Monomereinheiten, welche sich zur Bildung des Acrylatcopolymers während der Polymerisationsreaktion zusammenschließen, bei beiden Polymerisationsreaktionen identisch ist. Beispielsweise würde ein Acrylatcopolymer, welches aus kurzkettigen Monomeren gebildet ist, nach derselben Polymerisationsreaktion zwangsweise leichter sein als ein Acrylatcopolymer, welches aus langkettigen Monomeren gebildet ist. Dies ist insbesondere bei industriellen Herstellungsverfahren vorteilhaft, deren Anlagen bestimmte Anforderungen an die Polymerisationsreaktion, insbesondere bezüglich einer Reaktionsgeschwindigkeit, stellen, wodurch ein Einstellen der Eigenschaften der hergestellten Copolymere durch ein Anpassen der Polymerisationsreaktion oft nicht möglich ist.

Ferner wird vorgeschlagen, dass das Acrylatcopolymer zu mindestens 50 Gew.-%, vorteilhaft zu mindestens 70 Gew.-%, besonders vorteilhaft zu mindestens 90 Gew.-% aus Acrylsäureestern besteht, deren molare Massen mindestens 100 g/mol, vorteilhaft mindestens 120 g/mol, betragen. Beispielsweise können die Acrylsäureester n-Butylacrylat, 2-Ethylhexylacrylat, Isobutylacrylat, Ethylacrylat und/oder Propylacrylat aufweisen. Hierdurch kann eine mittlere Molmassenverteilung des Acrylatcopolymers optimiert werden. Vorteilhaft beträgt eine mittlere Molmassenverteilung des Acrylatcopolymers 450.000 bis 900.000 g/mol, besonders vorteilhaft 500.000 bis 800.00 g/mol und besonders bevorzugt 600,000 bis 700.000 g/mol. Besonders vorteilhaft kann durch das Einstellen der mittleren Molmassenverteilung des Acrylatcopolymers eine Kohäsion und eine Viskosität der Klebstoffschicht optimiert werden, insbesondere lässt sich der Klebstoff zur Herstellung der Klebstoffschicht mittels gängiger Beschichtungsverfahren, wie beispielsweise dem Vorhangbeschichten, dem Rakelbeschichten oder dem Siebdruck, beschichten. Denkbar wäre auch, dass der Klebstoff mittels Tintenstrahldruckverfahren oder 3D-Druckverfahren aufgetragen werden könnte.

Darüber hinaus wird vorgeschlagen, dass die Klebstoffschicht 5 bis 30 Gew.-%, vorteilhaft 10 bis 25 Gew.-%, besonders vorteilhaft 15 bis 20 Gew.-% an elektrisch leitenden Partikeln aufweist. Tests haben ergeben, dass das Hinzufügen von elektrisch leitenden Partikeln in der angegebenen Menge eine ausreichende elektrische Leitfähigkeit bereitstellt, ohne dass die Langzeithaftung der Klebstoffschicht beeinträchtigt wird.

Es wäre denkbar, dass die elektrisch leitenden Partikel als Metallpartikel oder metallbeschichtete Partikel ausgebildet sind. Um eine Konstruktion der Trockenelektrodenvorrichtung weiter zu verbessern, wird vorgeschlagen, dass die elektrisch leitenden Partikel als Kohlenstoffpartikel ausgebildet sind. Insbesondere können die elektrisch leitenden Partikel als Graphitpartikel oder Graphenpartikel ausgebildet sein. Hierdurch kann eine ausreichende elektrische Leitfähigkeit der Klebstoffschicht bei möglichst niedrigen Mengen an elektrisch leitenden Partikeln erreicht werden. Tests haben gezeigt, dass elektrisch Leitende Partikel, welche Metalle enthalten, entweder keine ausreichende elektrische Leitfähigkeit bereitstellen, oder in so großen Mengen hinzugefügt werden müssen, dass die Langzeithaftung der Klebstoffschicht reduziert wird. Ausschließlich Kohlenstoffpartikel haben Resultate erbracht, welche die Anforderungen bezüglich der Signalqualität und Langzeithaftung an die Trockenelektrodenvorrichtung erfüllen.

Prinzipiell könnten die elektrisch leitenden Partikel-beliebige Formen und Größen aufweisen, beispielsweise könnten sie sphärisch, stabförmig oder amorph sein. Um eine Konstruktion der Trockenelektrodenvorrichtung weiter zu verbessern, wird vorgeschlagen, dass die elektrisch leitenden Partikel als Mikroplättchen ausgebildet sind. Unter "Mikroplättchen" sollen schichtförmige Partikel verstanden werden, deren durchschnittliche Durchmesser mindestens 1 µm, vorteilhaft mindestens 3 µm, und höchstens 20 µm, vorteilhaft höchstens 10 µm, beträgt. Der durchschnittliche Durchmesser, auch "lateral size" genannt, bezeichnet eine Herstellerangabe, welche die durchschnittliche Partikelgröße definiert. Vorteilhaft beträgt eine Dicke der Mikroplättchen mindestens 1 nm, besonders vorteilhaft mindestens 5 nm, bevorzugt höchstens 500 nm, besonders bevorzugt höchstens 250 nm. Hierdurch kann eine ausreichende elektrische Leitfähigkeit, Biokompatibilität und Langzeithaftung der Trockenelektrodenvorrichtung gewährleistet werden. Vorteilhaft kann durch die geringe Dicke und große Fläche der Mikroplättchen ein hoher Einfluss auf die elektrische Leitfähigkeit der Klebstoffschicht pro elektrisch leitendem Partikel gewährleistet werden, wodurch die Menge an elektrisch leitenden Partikeln, die hinzugefügt werden muss, gering gehalten wird. Besonders vorteilhaft kann auf die Verwendung von Nanopartikeln, deren Erstreckung in jede Richtung im Nanometerbereich ist und damit in der Lage sind, in menschliche Zellen einzudringen, verzichtet werden.

Zusätzlich wird vorgeschlagen, dass eine Dicke der Klebstoffschicht, insbesondere in einem Bereich außerhalb des Kontaktelements, 10 bis 100 µm, vorteilhaft 20 bis 80 µm, besonders vorteilhaft 30 bis 60 µm, beträgt Hierdurch kann eine Konstruktion der Trockenelektrodenvorrichtung weiter verbessert werden. Tests haben ergeben, dass eine Dicke der Klebstoffschicht in dem genannten Wertebereich optimale Eigenschaften bezüglich einer elektrischen Leitfähigkeit und Langzeithaftung ermöglicht Klebstoffschichten, die zu dünn sind, haben den Nachteil, dass sie leicht reißen und somit einerseits schwer zu verarbeiten sind und andererseits keine robuste Haftung bereitstellen können. Klebstoffschichten, die zu dick sind, haben den Nachteil, dass sie höhere Materialkosten sowohl bezüglich des Klebstoffs als auch bezüglich der elektrisch leitenden Partikel verursachen und aufgrund ihrer Dicke zu kohäsiven Brüchen neigen, ferner müssten für eine ausreichende elektrische Leitfähigkeit so viele elektrisch leitende Partikel hinzugefügt werden, dass die Langzeithaftung der Klebstoffschicht darunter leiden

Ferner wird vorgeschlagen, dass eine Glasübergangslernperatur der Klebstoffschicht -50 bis -20°C beträgt. Tests haben ergeben, dass eine Klebstoffschicht, welche eine Glas- . übergangstemperatur in dem genannten Wertebereich aufweist, optimale Eigenschaften bezüglich einer Kohäsion bereitstellen kann. Vorzugsweise weist die Klebstoffschicht ein Speichermodul von höchstens 0,1 MPa auf und erfüllt damit das Dahlquist-Kriterirum. Hierdurch kann eine ausreichende Benetzung der Haut durch die Klebstoffschicht gewährleistet und eine Langzeithaftung der Trockenelektrodenvorrichtung verbessert werden.

Es wäre denkbar, dass die Klebstoffschicht mehr als 10 Gew.-% eines Vernetzers aufweist, um die Kohäsion der Klebstoffschicht zu optimieren. Um eine Konstruktion der Trockenelektrodenvorrichtung weiter zu verbessern, wird vorgeschlagen, dass die Klebstoffschicht 0,01 bis 10 Gew.-%, vorteilhaft 0,1 bis 5 Gew.-%, besonders vorteilhaft 0,1 5 bis 1 Gew.-% an Vernetzern aufweist. Unter einem "Vernetzer" soll eine Substanz verstanden werden, welche dazu vorgesehen ist, nach der Polymerisationsreaktion einzelne Acrylatcopolymere zur Bildung des Polymer-Netzwerks miteinander zu verknüpfen. Insbesondere unterscheidet sich der Vernetzer von einem "Härter", welcher dazu vorgesehen ist, während der Polymerisationsreaktion die Acrylatmonomere zu den Acrylatcopolymer zu verknüpfen. Insbesondere kann der Vernetzer vor, während oder bevorzugt nach einer Beschichtung der Klebstoffschicht hinzugefügt werden. Der Vernetzer kann insbesondere ein beliebiger bekannter, für Acrylatklebstoffe geeigneter, Vernetzer sein, wie beispielsweise Ethylenglycoldiacrylat, N,N'-Methylenbisacrylamid oder Aluminiumacetylacetonat. Es wäre denkbar, dass die Klebstoffschicht mehrere verschiedene Vernetzer aufweist. Vorzugsweise weist die Klebstoffschicht genau einen Vernetzer auf. Hierdurch kann eine Langzeithaftung der Trockenelektrodenvorrichtung verbessert werden. Vorteilhaft kann insbesondere durch das Einstellen der Kohäsion mittels der Glasübergangstemperatur und der mittleren Molmassenverteilung des Acrylatcopolymers darauf verzichtet werden, die Kohäsion stattdessen durch Zugabe von Vernetzern einzustellen. Besonders vorteilhaft kann durch eine Reduzierung der benötigten Menge an Vernetzern eine Adhäsion der Klebstoffschicht verbessert werden, da durch den Vernetzer eine Anzahl freier Copolymerketten, welche zum Aufbau einer Haftverbindung mit der Haut beitragen, reduziert wird.

In einem weiteren Aspekt der Erfindung wird vorgeschlagen, dass die Klebstoffschichteinheit eine erste Klebstoffschicht und eine zweite Klebstoffschicht aufweist. Vorteilhaft ist die erste Klebstoffschicht unmittelbar an der Anschlusseinheit, insbesondere dem Kontaktelement, angeordnet. Besonders vorteilhaft ist die zweite Klebstoffschicht in dem Anwendungszustand unmittelbar an der Haut angeordnet. Insbesondere weisen die erste Klebstoffschicht und die zweite Klebstoffschicht jeweils einen elektrisch leitenden Klebstoff auf. Vorzugsweise sind die erste Klebstoffschicht und die zweite Klebstoffschicht frei von strukturellen Klebstoffen, welche dazu vorgesehen sind, nach einem Applizieren auszuhärten, wobei die strukturellen Klebstoffe vor dem Aushärten keine oder nur eine geringe Haftung aufweisen. Insbesondere weisen die erste Klebstoffschicht und die zweite Klebstoffschicht Haftklebstoffe auf. Beispielsweise können die erste Klebstoffschicht und die zweite Klebstoffschicht jeweils einen Synthesekautschuk-Haftklebstoff einen Naturkautschuk-Haftklebstoff, einen Acrylat-Haftklebstoff, einen Polyurethan-Haftklebstoff oder einen Silikon-Haftklebstoff aufweisen. Es wäre denkbar, dass die erste Klebstoffschicht und die zweite Klebstoffschicht bezüglich eines Klebstoffs identisch zueinander sind. Vorteilhaft sind die erste Klebstoffschicht und die zweite Klebstoffschicht bezüglich zumindest eines Klebstoffs voneinander verschieden ausgebildet. Prinzipiell könnten die erste Klebstoffschicht und die zweite Klebstoffschicht zueinander identische Dicken aufweisen, alternativ könnten die erste Klebstoffschicht und die zweite Klebstoffschicht voneinander verschiedene Dicken aufweisen. Denkbar wäre, dass sich bei der Draufsicht eine Kontur der ersten Klebstoffschicht bezüglich einer Form von einer Kontur der zweiten Klebstoffschicht unterscheidet, bevorzugt sind in der Draufsicht die Kontur der ersten Klebstoffschicht und die Kontur der zweiten Klebstoffschicht zueinander formgleich. Bevorzugt weist die zweite Klebstoffschicht einen biokompatiblen Klebstoff auf. Hierdurch kann eine Konstruktion der Trockenelektrodenvorrichtung, insbesondere hinsichtlich einer Designfreiheit, verbessert werden. Vorteilhaft kann im Vergleich zu einer Ausgestaltung der Klebstoffcchichteinheit mit nur einer Klebstoffschicht ein erweitertes Spektrum von Anforderungen erfüllt werden. So kann es beispielsweise bei Anwendungen auf empfindlicher und/oder dünner Haut erwünscht sein, die Klebekraft zu reduzieren, um Hautirritationen und/oder Hautschäden zu vermeiden. Allerdings ist es auch bei diesen Anwendungen weiterhin erforderlich, eine robuste Fixierung der Anschlusseinheit zu gewährleisten, um die Signalqualität zu erhalten. Entsprechend ist es bei solchen Anwendungen nicht möglich, sämtliche Anforderungen mittels einer einzigen Klebstoffschicht zu erfüllen.

Um eine Konstruktion der Trockenelektrodenvorrichtung noch weiter zu verbessern, wird vorgeschlagen, dass die erste Klebstoffschicht eine Klebkraft auf Metall, insbesondere Stahl, gemäß DIN EN 1939 (Dez 2003) in Höhe von mindestens 10 N/25 mm, vorteilhaft mindestens 15 N/25 mm und besonders vorteilhaft mindestens 25. N/25 mm und die zweite Klebstoffschicht eine Klebkraft auf Haut gemäß DIN EN 1939 (Dez 2003) in Höhe von mindestens 1 N/25 mm, vorteilhaft mindestens 2 N/25 mm und besonders vorteilhaft mindestens 5 N/25 mm aufweist. Hierdurch kann gleichzeitig eine Signalqualität verbessert und Hautirritationen und/oder Hautschäden bei einer Entfernung der Trockenelektrodenvorrichtung vermieden werden.

Die Klebkraft in gemäß DIN EN 1939 (Dez 2003) ist als die Kraft, die erforderlich ist, um einen Klebbandstreifen von einem bestimmten Haftgrund unter einem bestimmten Winkel und mit einer bestimmten Geschwindigkeit abzuziehen. Die Messung der Klebkraft der Klebstoffschichten gemäß DIN EN 1939 (Dez 2003) wird wie folgt durchgeführt: Es wird eine Probe der Klebstoffschicht mit einer Breite von 25 mm und einer Länge von ca. 100 mm hergestellt. Anschließend wird die Probe auf ein Substrat appliziert, sodass sich eine Verklebungsfläche von 625 mm² ergibt. Im Fall der ersten Klebstoffschicht besteht das Substrat aus Stahl, im Fall der zweiten Klebstoffschicht ist das Substrat ein Haut-Imitat und kann aus einem Polyurethan-Leder oder einer Kunsthaut bestehen. Auf eine dem Substrat gegenüberliegenden Seite der Probe wird ein ca. 10 cm langer Streifen einer 50 µm dicken PET-Folie appliziert. Anschließend wird die Probe mittels einer 5 kg Anrollwalze an das Substrat gepresst, um eine vollständige Klebung u gewährleisten. Nach 10 Minuten wird die Probe mit einer Geschwindigkeit von 300 mm/min und unter einem Abzugswinkel von 180° von dem Substrat abgezogen, wobei die zur vollständigen Entfernung der Probe notwendige Kraft gemessen wird. Der Mittelwert aus fünf Einzelmessungen wird als Klebkraft in N/25mm angegeben.

Die Messung findet unter Normbedingungen statt, welche eine Temperatur von 23°C ± 2°C und eine Luftfeuchtigkeit von 50 % ± 5 % definieren.

Es wäre denkbar, dass die erste Klebstoffschicht und die zweite Klebstoffschicht direkt aufeinander laminiert sind. Bevorzugt weist die Klebstoffschichteinheit einen elektrisch leitenden Träger auf, welches zwischen der ersten Klebstoffschicht und der zweiten Klebstoffschicht angeordnet ist. Es wäre möglich, dass der elektrisch leitende Träger als ein Textil ausgebildet ist und beispielsweise aus Metallfasern, beschichteten Glasfasern, beschichteten Kunststofffasern und/oder elektrisch leitenden Polymerfasern bestehen könnte. Vorzugweise ist der elektrisch leitende Träger frei von Ausnehmungen und beispielsweise als eine Metallfolie, eine elektrisch leitende Polymerfolie oder eine Kohlenstofffolie ausgebildet. Hierdurch kann eine Designfreiheit noch weiter gesteigert werden. Vorteilhaft können für die erste Klebstoffschicht und die zweite Klebstoffschicht Klebstoffe verwendet werden, welche keine gute Haftung aufeinander aufweisen. Besonders vorteilhaft können für die erste Klebstoffschicht auch Klebstoffe verwendet werden, welche nicht biokompatibel sind, solange der elektrisch leitende Träger eine ausreichende Barriere darstellt, um ein Diffundieren des Klebstoffs zu verhindern.

Prinzipiell könnten die erste Klebstoffschicht und die zweite Klebstoffschicht bezüglich einer Form und Größe zueinander identisch ausgebildet sein. Um eine Konstruktion der Trockenelektrodenvorrichtung noch weiter zu verbessern, wird vorgeschlagen, dass in der Draufsicht eine Konturfläche der zweiten Klebstoffschicht mindestens 25 %, vorteilhaft mindestens 50 %, größer ist als eine Konturfläche der ersten Klebstoffschicht. Insbesondere stellt der elektrisch leitende Träger eine Vergrößerung der Kontaktfläche bereit, wobei in der Draufsicht eine Kontur der Kontaktfläche identisch zu der Kontur der zweiten Klebstoffschicht ist. definiert die zweite Klebstoffschicht eine Größe der Kontaktfläche. Vorteilhaft sind die Kontur der ersten Klebstoffschicht und die Kontur der zweiten Klebstoffschicht zueinander konzentrisch angeordnet. Besonders vorteilhaft beträgt bei der Draufsicht ein minimaler Abstand einer Kontur der zweiten Klebstoffschicht von einer Kontur der ersten Klebstoffschicht an jeder Stelle mindestens 1 mm, vorteilhaft mindestens 2 mm. Hierdurch kann ein asymmetrischer Aufbau der Trockenelektrodenvorrichtung bereitgestellt werden, welcher sicherstellt, dass bei einer Verwendung von nicht biokompatiblen Klebstoffen in der ersten Klebstoffschicht ein ausreichender Abstand eines Rands der ersten Klebstoffschicht zu einem Rand der zweiten Klebstoffschicht vorhanden ist, um ein Diffundieren des Klebstoffs der ersten Klebstoffschicht zu verhindern.

Weitere Vorteile ergeben sich aus der folgenden Zeichnungsbeschreibung.

Es zeigen: .
- Fig. 1: eine schematische Darstellung einer Trockenelektrodenvorrichtung in einer Querschnittsansicht,
- Fig. 2: eine schematische Darstellung der Trockenelektrodenvorrichtung in einer Draufsicht von oben,
- Fig. 3: eine schematische Darstellung eines weiteren Ausführungsbeispiels einer Trockenelektrodenvorrichtung in einer Querschnittsansicht,
- Fig. 4: eine schematische Darstellung der Trockenelektrodenvorrichtung aus Fig. 3 in einer Draufsicht von oben,
- Fig. 5: eine schematische Darstellung eines weiteren Ausführungsbeispiels einer Trockenelektrodenvorrichtung in einer Querschnittsansicht und
- Fig. 6: eine schematische Darstellung der Trockenelektrodenvorrichtung aus Fig. 5 in einer Draufsicht von oben.

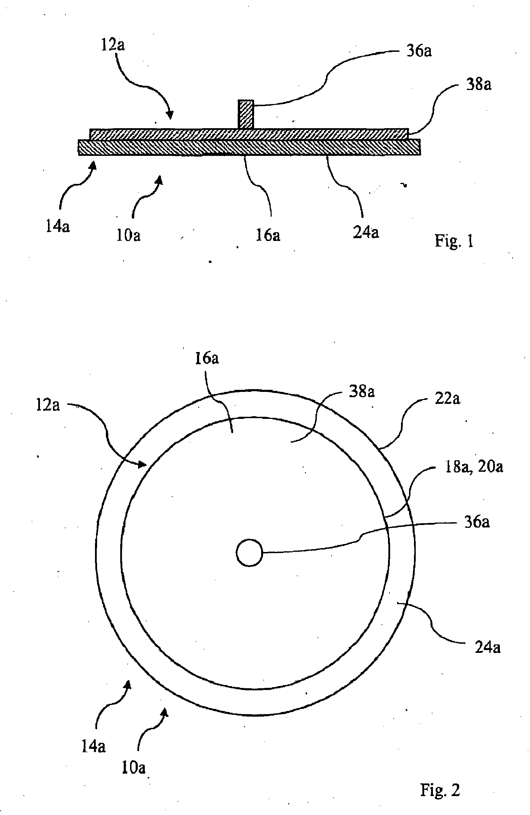

Figuren 1 und 2 zeigen eine Trpckcnclcktrodenvorrichtung 10a. Die Trockenelektrodenvorrichtung 10a ist als eine einzelne Einweg-Trockenelektrode ausgebildet, welche dazu vorgesehen ist, in einem Anwendungszustand auf eine Haut aufgebracht zu werden, ein EKG-Signal zu messen und die erfassten Daten an eine externe Einheit zur Auswertung zu übertragen. Alternativ könnte die Trockenelektrodenvorrichtung 10a auch Teil eines Trockenelektrodensystems (nicht dargestellt) sein, welches mehrere Trockenelektrodenvorrichtungen aufweist. Weiterhin könnte die Trockenelektrodenvorrichtung 10a zusätzlich oder alternativ zu dem EKG-Signal auch andere Biosignale messen. Die Trockenelektrodenvorrichtung 10a ist dazu vorgesehen, das EKG-Signal anhand einer direkten elektrischen Kopplung zu messen, alternativ könnte die Trockenelektrodenvorrichtung 10a dazu vorgesehen sein, das EKG-Signal anhand einer indirekten, kapazitiven elektrischen Kopplung zu messen.

Die Trockenelektrodenvorrichtung 10a weist eine elektrisch leitende Anschlusseinheit 12a auf. Die Anschlusseinheit 12a besteht aus Edelstahl. Alternativ könnte die Anschlusseinheit 12a auch aus einem Kunststoff bestehen, welcher mit elektrisch leitenden Materialien beschichtet ist oder elektrisch leitende Partikel aufweist. Die Anschlusseinheit 12a weist ein Anschlusselement 36a auf. Das Anschlusselement 36a ist als ein Druckknopfelement ausgebildet und dazu vorgesehen, in dem Anwendungszustand eine elektrisch leitende Rastverbindung mit der externen Einheit auszubilden. Alternativ könnte das Anschlusselement 36a als ein beliebiges anderes Verbindungselement ausgebildet sein, wie beispielsweise ein Klammerelement oder ein Gewindeelement.

Die Anschlusseinheit 12a weist ein Kontaktelement 38a auf. Das Kontaktelement 38a ist als eine Kontaktplatte ausgebildet. Das Kontaktelement 38a und das Anschlusselement 36a sind als ein zusammenhängendes Bauteil ausgebildet. Alternativ könnten das Kontaktelement 38a und das Anschlusselement 36a mittels beliebiger Verbindungen miteinander verbunden sein, wie beispielsweise einer Nietverbindung, Klebeverbindung, Schweißverbindung, Rastverbindung oder Drehverbindung. Insbesondere können das Kontaktelement 38a und das Anschlusselement 38a in einer alternativen Ausgestaltung gemeinsam ein Druckknopfelement ausbilden.

Die Trockenelektrodenvorrichtung 10a weist eine elektrisch leitende Klebstoffschichteinheit 14a auf. Die elektrisch leitende Klebstoffschichteinheit 14a weist genau eine Klebstoffschicht 24a auf. Alternativ könnte die elektrisch leitende Klebstoffschichteinheit 14a eine beliebige Anzahl weiterer Klebstoffschichten aufweisen. Die Klebstoffschichteinheit 14a ist unmittelbar an der Anschlusseinheit 12a angeordnet. Das Kontaktelement 38a liegt auf der Klebstoffschicht 24a auf. Alternativ könnte das Kontaktelement 38a teilweise oder vollständig in die Klebstoffschicht 24a eingebettet sein.

Die Klebstoffschichteinheit 14a weist eine Kontaktfläche 16a zur Kontaktierung der Haut auf. Die Kontaktfläche 16a ist eine Teiloberfläche einer der Anschlusseinheit 12a abgewandten Oberfläche der Klebstoffschichteinheit 14a. Die Klebstoffschichteinheit 14a stellt in dem Anwendungszustand eine elektrische Kopplung zwischen der Anschlusseinheit und der Haut bereit. Die elektrische Kopplung ist eine direkte elektrische Leitung durch die Klebstoffschicht 24a in Bereich der Kontaktfläche 16a.

Die Klebstoffschichteinheit 14a stellt in dem Anwendungszustand eine Fixierung der Trockenelektrodenvorrichtung 10a relativ zu der Haut vollständig bereit. Alternativ könnte die Trockenelektrodenvorrichtung 10a zusätzlich eine Abdeckeinheit (nicht dargestellt) aufweisen, welche aus einem Textil und einem Hautklebstoff besteht und sich über die Klebstoffschichteinheit 14a hinaus erstreckt, um in dem Anwendungszustand zu einer Fixierung der Trockenelektrodenvorrichtung 10a relativ zu der Haut beizutragen. Die Trockenelektrodenvorrichtung 10a ist in einem Anwendungszustand dargestellt. Die Trockenelektrodenvorrichtung 10a weist in einem nicht applizierten Zustand einen Release Liner (nicht dargestellt) auf, welcher eine der Anschlusseinheit 12a abgewandte Seite der Klebstoffschichteinheit 14a abdeckt.

Figur 2 zeigt die Trockenelektrodenvorrichtung 10a in einer Draufsicht von oben. In der Draufsicht sind die Konturen der Elemente und Einheiten unabhängig von über oder unter den jeweiligen Elementen und Einheiten angeordneten weiteren Elementen und Einheiten dargestellt, entsprechend sind die Konturen unabhängig davon, ob die Draufsicht von oben oder unten erfolgt. Eine Kontur 22a der Klebstoffschichteinheit 14a und eine Kontur 20a der Anschlusseinheit 12a sind zueinander formidentisch und konzentrisch ausgebildet. Die Kontur 22a der Klebstoffschichteinheit 14a und die Kontur 20a der Anschlusseinheit 12a sind kreisförmig ausgebildet, alternativ könnten die Kontur 22a der Klebstoffschichteinheit 14a und die Kontur 20a der Anschlusseinheit 12a beliebige andere Formen, wie beispielsweise Rechtecke oder Ovale, aufweisen. Die Kontur 22a der Klebstoffschichteinheit 14a umschließt die Kontur 20a der Anschlusseinheit 12a, wobei ein minimaler Abstand der Kontur 22a der Klebstoffschichteinheit 14a von der Kontur 20a der Anschlusseinheit 12a an jedem Punkt 2 mm ist. Eine Kontur 18a der Kontaktfläche 16a ist identisch zur Kontur 20a der Anschlusseinheit 12a, entsprechend überlappt eine Konturfläche der Anschlusseinheit 12a eine Konturfläche der Kontaktfläche 16a zu 100%. Alternativ könnte die Klebstoffschicht 24a auch isotrop leitend sein oder die Trockenelektrodenvorrichtung 10a eine elektrisch leitende Schicht zwischen der Klebstoffschichteinheit 13a und der Anschlusseinheit 12a aufweisen. In diesem Fall wäre die Kontur 18a der Kontaktfläche 16a identisch zur Kontur 22a der Klebstoffschichteinheit 14a, wodurch die Konturfläche der Anschlusseinheit 12a kleiner als die Konturfläche der Kontaktfläche 16a wäre und selbige nur zu etwa 83 % überlappen würde, da eine Konturfläche der Klebstoffschichteinheit 14a etwa 17 % größer als die Konturfläche der Anschlusseinheit 12a ist.

Die Klebstoffschicht 24a weist ein Polymer-Netzwerk mit zumindest einem Acrylatcopolymer und zumindest eine Art von elektrisch leitenden Partikeln auf. Alternativ wäre es denkbar, dass die Klebstoffschicht 24a einen elektrisch leitenden Silikon-Haftklebstoff und/oder einen elektrisch leitenden Kautschuk-Haftklebstoff aufweisen könnte. Das Acrylatcopolymer weist 95 Gew.-% an unpolaren Acrylatmonomeren und 5 Gew.-% an polaren Acrylatmonomeren auf. Das Acrylatcopolymer weist 5 Gew.-% an Acrylsäure auf. Das Acrylatcopolymer besteht zu 95°Gew.-% aus Acrylsäureestern, deren molare Massen mindestens 100 g/mol betragen. Eine mittlere Molmassenverteilung des Acrylatcopolymers beträgt 650.000 g/mol.

Die Klebstoffschicht 24a ist anisotrop elektrisch leitend. Die Klebstoffschicht 24a weist 17,5 Gew.-% an elektrisch leitenden Partikeln auf. Die elektrisch leitenden Partikel sind als Graphen-Mikroplättchen ausgebildet. Es wäre auch denkbar, dass die elektrisch leitenden Partikel als Metall-Mikroplättchen oder Kohlenstoff-Mikrokugeln ausgebildet sein könnten. Die Graphen-Mikroplättchen weisen laut Hersteller einen mittleren Durchmesser von 5 µm und eine Dicke von unter 50 nm auf.

Die Klebstoffschicht 24a weist eine Dicke von 30 µm auf. Eine Glasübergangstemperatur der Klebstoffschicht 24a beträgt -47°C. Die Klebstoffschicht 24a weist 0,2 Gew.-% an Vemetzem und 99,8 Gew.-% an Substanzen des Acrylatcopolymers auf. In der folgenden Tabelle ist eine Zusammensetzung der Klebstoffschicht 24a dargestellt:

| Substanz | Funktion | Gewichtsanteil |
|---|---|---|
| n-Butylacrylat | Unpolares Acrylatmonomer | 39,9 Gew.-% |
| 2-Ethylhexylacrylat | Unpolares Acrylatmonomer | 54,9 Gew.-% |
| Acrylsäure | Polares Acrylatmonomer | 5 Gew.-% |
| Aluminiumacetylacetonat | Vernetzer | 0,2 Gew.-% |

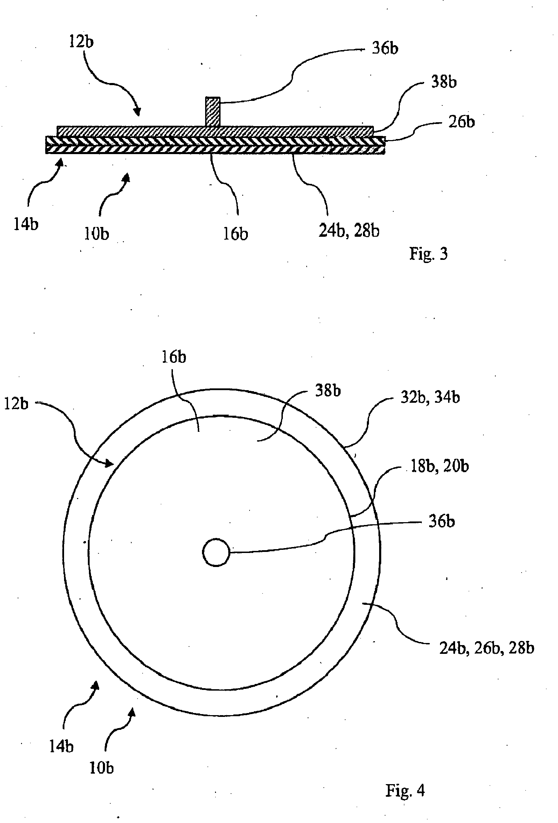

Die Figuren 3 und 4 zeigen eine alternative Ausgestaltung einer Trockenelektrodenvorrichtung 10b. Im Folgenden werden lediglich Unterschiede zwischen dieser Trockenelektrodenvorrichtung 10b und der Trockenelektrodenvorrichtung 10a des vorherigen Ausführungsbeispiels, insbesondere der Figuren 1 und 2, aufgezählt.

Die Trockenelektrodenvorrichtung 10b weist eine Klebstoffschichteinheit 14b auf. Die Klebstoffschichteinheit 14b weist eine erste Klebstoffschicht 26b und eine zweite Klebstoffschicht 28b auf. Die erste Klebstoffschicht 26b und die zweite Klebstoffschicht 28b weisen jeweils eine Dicke von 15 µm auf. Die erste Klebstoffschicht 26b ist unmittelbar an einer Anschlusseinheit 12b angeordnet. Die zweite Klebstoffschicht 28b ist in dem Anwendungszustand unmittelbar an der Haut angeordnet. Die erste Klebstoffschicht 26b weist eine Klebkraft auf Metall gemäß DIN EN 1939 (Dez 2003) in Höhe von mindestens 10 N/25 mm auf. Die erste Klebstoffschicht 26b weist einen elektrisch leitenden Polyurethan-Haftklebstoff auf. Alternativ könnte die erste Klebstoffschicht 26b einen beliebigen anderen elektrisch leitenden Haftklebstoff, welcher dazu vorgesehen ist, auf Metalloberflächen verwendet zu werden, aufweisen.

Die zweite Klebstoffschicht 28b weist eine Klebkraft auf Haut gemäß DIN EN 1939 (Dez 2003) in Höhe von mindestens 2 N/25 mm auf. Die zweite Klebstoffschicht 28b ist bis auf eine Dicke identisch zur Klebstoffschicht 24a der Figuren 1 und 2, weshalb sie in den Figuren 3 und 4 sowohl das Bezugszeichen 24b als auch das Bezugszeichen 28b aufweist. Alternativ könnte die zweite Klebstoffschicht 26b einen beliebigen anderen elektrischen leitenden Haftklebstoff, welcher dazu vorgesehen ist, auf Hautoberflächen verwendet zu werden, aufweisen.

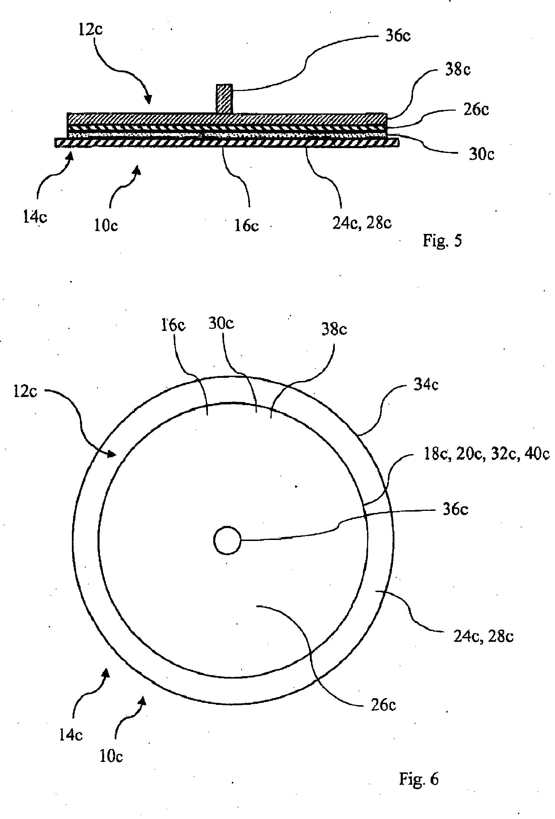

Die Figuren 5 und 6 zeigen eine alternative Ausgestaltung einer Trockenelektrodenvorrichtung 10c. Im Folgenden werden lediglich Unterschiede zwischen dieser Trockenelektrodenvorrichtung 10c und der Trockenelektrodenvorrichtung 10b der vorherigen Ausführungsbeispiele, insbesondere der Figuren 3 und 4, aufgezählt.

Die Trockenelektrodenvorrichtung 10c weist eine Klebstoffschichteinheit 14c auf. Die Klebstoffschichteinheit 14c weist einen elektrisch leitenden Träger 30c auf. Der elektrisch leitende Träger 30c ist zwischen der ersten Klebstoffschicht 26c und der zweiten Klebstoffschicht 28c angeordnet. Der elektrisch leitende Träger 30c ist als eine Aluminiumfolie ausgebildet. Alternativ könnte der elektrisch leitende Träger 30c auch als eine beliebige andere elektrisch leitende Schicht ausgebildet sein. In der Draufsicht ist eine Konturfläche der zweiten Klebstoffschicht 28c etwa 30 % größer als eine Konturfläche der ersten Klebstoffschicht 26c. Eine Kontur 40c des elektrisch leitenden Trägers 30c ist identisch zu einer Kontur 18c einer Kontaktfläche 16a der Klebstoffschichteinheit 14c, einer Kontur 20c einer Anschlusseinheit 12c und einer Kontur 32c der ersten Klebstoffschicht 26c. Eine Kontur 34c der zweiten Klebstoffschicht 28c umschließt die Kontur 40c des elektrisch leitenden Trägers 30c.

### Bezugszeichen

- 10: Trockenelektrodenvorrichtung
- 12: Anschlusseinheit
- 14: Klebstoffschichteinheit
- 16: Kontaktfläche
- 18: Kontur der Kontaktfläche
- 20: Kontur der Anschlusseinheit
- 22: Kontur der Klebstoffschichteinheit
- 24: Klebstoffschicht
- 26: Erste Klebstoffschicht
- 28: Zweite Klebstoffschicht
- 30: Träger
- 32: Kontur der ersten Klebstoffschicht
- 34: Kontur der zweiten Klebstoffschicht
- 36: Anschlusselement
- 38: Kontaktelement
- 40: Kontur des Trägers

## Patentansprüche

1. Trockenelektrodenvorrichtung (10a-c) mit zumindest einer elektrisch leitenden Anschlusseinheit (12a-c) und mit einer, insbesondere elektrisch leitenden, Klebstoffschichteinheit (14a-c), welche eine Kontaktfläche (16a-c) zur Kontaktierung einer Haut aufweist und welche in einem Anwendungszustand zur Bereitstellung einer elektrischen Energieübertragung zwischen der Anschlusseinheit (12a-c) und der Haut vorgesehen ist, **dadurch gekennzeichnet, dass** bei einer Draufsicht eine Konturfläche der Anschlusseinheit (12a-c) eine Konturfläche der Kontaktfläche (16a-c) zumindest zu 80 % überlappt.

2. Trockenelektrodenvorrichtung (10a-c) nach Anspruch 1, **dadurch gekennzeichnet, dass** die Klebstoffschichteinheit (14a-c) unmittelbar an der Anschlusseinheit (12a-c) angeordnet ist.

3. Trockenelektrodenvorrichtung (10a-c) nach Anspruch 1 oder 2, **dadurch gekennzeichnet, dass** bei der Draufsicht eine Konturfläche der Klebstoffschichteinheit (14a-c) höchstens 100 % größer als die Konturfläche der Anschlusseinheit (12a-c) ist.

4. Trockenelektrodenvorrichtung (10a-c) nach einem der vorhergehenden Ansprüche, **dadurch gekennzeichnet, dass** die Klebstoffschichteinheit (14a-c) zumindest eine Klebstoffschicht (24a-c) aufweist, welche ein Polymer-Netzwerk mit zumindest einem Acrylatcopolymer und zumindest eine Art von elektrisch leitenden Partikeln aufweist und welche frei von ionischen Flüssigkeiten ist.

5. Trockenelektrodenvorrichtung (10a-c) nach Anspruch 4, **dadurch gekennzeichnet, dass** das Acrylatcopolymer 70 bis 98,5 Gew.-% an unpolaren Acrylatmonomeren und 1,5 bis 30 Gew.-% an polaren Acrylatmonomeren aufweist.

6. Trockenelektrodenvorrichtung (10a-c) nach Anspruch 4 oder 5, **dadurch gekennzeichnet, dass** das Acrylatcopolymer 0,1 bis 10 Gew.-% an Acrylsäure aufweist.

7. Trockenelektrodenvorrichtung (10a-c) nach einem der Ansprüche 4 bis 6, **dadurch gekennzeichnet, dass** das Acrylatcopolymer zu mindestens 50°Gew.-% aus Acrylsäureestern besteht, deren molare Massen mindestens 100 g/mol betragen.

8. Trockenelektrodenvorrichtung (10a-c) nach einem der Ansprüche 4 bis 7, **dadurch gekennzeichnet, dass** die Klebstoffschicht (24a-c) 5 bis 30 Gew.-% an elektrisch leitenden Partikeln aufweist.

9. Trockenelektrodenvorrichtung (10a-c) nach einem der Ansprüche 4 bis 8, **dadurch gekennzeichnet, dass** die elektrisch leitenden Partikel als Kohlenstoffpartikel ausgebildet sind.

10. Trockenelektrodenvorrichtung (10a-c) nach einem der Ansprüche 4 bis 9, **dadurch gekennzeichnet, dass** die elektrisch leitenden Partikel als Mikroplättchen ausgebildet sind.

11. Trockenelektrodenvorrichtung (10a-c) nach einem der Ansprüche 4 bis 10, **dadurch gekennzeichnet, dass** eine Dicke der Klebstoffschicht (24a-c) 10 bis 100 µm beträgt.

12. Trockenelektrodenvorrichtung (10a-c) nach einem der Ansprüche 4 bis 11, **dadurch gekennzeichnet, dass** die Klebstoffschicht (24a-c) 0,01 - 10 Gew.- % an Vernetzern aufweist.

13. Trockenelektrodenvorrichtung (10b-c) nach einem der vorhergehenden Ansprüche, **dadurch gekennzeichnet, dass** die Klebstoffschichteinheit (14b-c) eine erste Klebstoffschicht (26b-c) und eine zweite Klebstoffschicht (28b-c) aufweist.

14. Trockenelektrodenvorrichtung (10c) nach Anspruch 13, **dadurch gekennzeichnet, dass** die Klebstoffschichteinheit (14c) einen elektrisch leitenden Träger (30c) aufweist, welcher zwischen der ersten Klebstoffschicht (26c) und der zweiten Klebstoffschicht (28c) angeordnet ist.

15. Trockenelektrodenvorrichtung (10c) nach Anspruch 14, **dadurch gekennzeichnet, dass** in der Draufsicht eine Konturfläche der zweiten Klebstoffschicht (28c) mindestens 25 % größer ist als eine Konturfläche der ersten Klebstoffschicht (26c).
